# EUROPEAN PATENT APPLICATION

(11) **EP 0 935 977 A2**
(43) Date of publication of application: **18.08.1999**
(21) Application number: 99101640.3
(22) Date of filing: 04.02.1999
(51) Int. Cl.: A61M 25/04, A61M 25/10, A61M 25/01, A61M 25/088

(54) **Urethral catheter and guide**

(30) Priority: 04.02.1998 US 18664
(71) Applicant: ARGOMED Ltd., 46766 Herzlia Pituach (IL)
(72) Inventor: Eshel, Uzi, Herzlia (IL); Lazarovitz, Jakob, Hod Hasharon (IL)
(74) Representative: Modiano, Guido, Dr.-Ing.

(57) **Abstract**

An indwelling catheter (10) for insertion into a patient's urinary tract is provided and includes (a) a first tubular member (12) having a distal end (14) and a proximal end (16); (b) a second tubular member (18) having a length, a distal end (20) and a proximal end (22), the first and second tubular members (12,18) having such a diameter for enabling drainage of physiological fluids therethrough; (c) a first inflatable balloon (24) being inflatably attached at a portion of the second tubular member (18); and (d) a first connecting tube (26) of substantially smaller diameter interconnecting the first and second tubular members (12,18) to form a gap of a known maximal length between the first and second tubular members (12,18), the first connecting tube (26) being in fluid communication with the first inflatable balloon (24), so as to enable inflating the first inflatable balloon (24) via the first connecting tube (26). The length of the second tubular member (18), the known maximal length of the gap and a location of the portion of the second tubular member (18) to which the first inflatable balloon (24) is inflatably attached are selected such that, when positioned in the urinary tract, the balloon (24) is anchored within the urinary bladder, so as to position the second tubular member (18) within the prostatic urethra with its proximal end (22) positioned distally, and in close proximity, to the sphincter, and with its distal end (20) protruding into the urinary bladder, and further so as to position the first tubular member (12) within the penile urethra with its distal end (14) positioned proximally, and in close proximity, to the sphincter, while the first connecting tube (26) traverses the sphincter, thereby permitting voluntary control of the sphincter.

## Description

This is a continuation-in-part of U.S. patent application 09/018,664, filed February 4, 1998, the specification of which is hereby incorporated by reference.

### FIELD AND BACKGROUND OF THE INVENTION

The present invention relates to an internal catheter for insertion into the urethra of a patient and to a catheter guide for effecting such insertion, More particularly, the present invention relates to an indwelling catheter specifically adapted for treating obstruction of the prostatic urethra and to a catheter guide, for flushing the patient's urinary tract and for the insertion and positioning of the catheter therewithin. The invention further relates to a method of inserting and positioning an indwelling catheter in the urinary tract of a patient using the guide of the present invention.

Benign prostate hyperplasia (BPH) is a condition wherein enlargement of the prostate gland constricts and blocks the portion of the urethra traversing the prostate (i.e., the prostatic urethra) and leads to difficulties in normal urination.

BPH is typically treated by using surgical procedures such as trans urethral resection of the prostate (TURP) or preferably by non-surgical procedures, such as thermal ablation of the prostate, a procedure which typically employs a catheter supplemented with a balloon head positioned within the prosthetic urethra, in which balloon heated water is recirculated.

Following a thermal ablation procedure, temporary blockage of the prostatic urethra is usually experienced due to extensive swelling and edema formation. Proper healing of the prostatic tissue and long-term urination of the patient requires the removal of the ablation balloon catheter and the subsequent insertion of a drainage catheter into the prostatic urethra.

Several types of drainage catheters are known. One type includes tubing leading from the bladder through the entire length of the urinary tract to the outside. Such catheters, beside being highly uncomfortable, suffer from two major limitations. First, such catheters do not allow voluntary urination. Second, upon prolonged installation, they frequently cause urinary tract infections.

Another type includes indwelling catheters which are typically used when long catheterizing periods are required, such as following BPH elimination treatment, during the healing of the tissues damaged by the treatment. During this time period the healing and scarring of the prostate tissue around the catheter ensures that the prostatic urethra remains dilated after the catheter is removed.

Presently, there exist various indwelling catheters for insertion into the patient's urinary tract for enabling effective drainage of fluids and tissue particles following a prostate unblocking procedure.

Examples of such indwelling catheters are disclosed in U.S. Pat. Nos. 3,811,450; 5,176,626; and 5,514,178.

An indwelling catheter employed in treating and unblocking of a prostatic urethra must meet several requirements. The catheter must have a portion which traverses the blocked prostatic urethra. The catheter must allow the patient control over urination, either biologically (voluntary sphincter control) or mechanically (e.g., a mechanical valve). In addition, an indwelling catheter must be appropriately positioned and anchored, such that no permanent movement of the device is experienced during service.

To meet the above requirements, presently employed indwelling catheters have incorporated several configurations of inflatable balloons, for anchoring and appropriately positioning the catheter within the patient's urinary tract. Such configurations typically include balloons, such as in Folley catheters, in which a positioning and anchoring balloon is positioned within the patient's bladder and is anchored against the inner-wall of the bladder opening, or ring type balloons which are inflated against the prostatic urethra and serve to both unblock the prostatic urethra and establish a tract for urination. Furthermore, balloons which are carried on the catheter itself, or alternatively on a catheter guide used for its insertion and positioning, serve for appropriately inserting and positioning the catheter by functioning as "insertion halters" and as "position reference" when halted by the patient's sphincter.

Other anchoring methods, as demonstrated by the transurethral bridge feature of the catheter produced by Boston Scientific (known as TRESTLE), a schematic depiction of which is shown in Figure 5, also exist.

In this configuration, the placement of two tubes, interconnected by a wire, on opposite sides of the sphincter anchors the catheter against longitudinal displacement, and also allows patient voluntary control over urination.

Limitations are inherent to the catheters of the above mentioned designs. For example, some catheters are constructed such that they traverse the sphincter region of the urethra, not allowing for sphincter closure and thus necessitating the use of a valved line for urination control. These catheters are further limited in such that the urination line which leads from the bladder to the outside environment is often the cause of urinary tract infections, which necessitate the removal of the catheter, followed by antibiotic treatment and repositioning of a new catheter, causing great discomfort to the patient. On the other hand, catheters employing transurethral bridges often tend to proximally displace within the urethra when the patient urinates, due to a relief in the anchoring of the catheter upon sphincter dilation.

Additionally, positioning of a catheter via a sphincter balloon can often be difficult and time consuming, whereas positioning of a catheter via a bladder balloon often necessitates a more complex guide and catheter system.

There is thus a widely recognized need for, and it would be highly advantageous to have, an indwelling urethral catheter devoid of the above limitations. The catheter according to the present invention can be used for drainage of fluids and tissue particles through the patient's prostatic urethra following a non-surgical medical procedure such as thermal ablation of the prostate, wherein a long-term indwelling catheter is needed, while allowing voluntary control over urination.

### SUMMARY OF THE INVENTION

According to one aspect of the invention described below, there is provided an indwelling catheter for insertion into a patient's urinary tract, the tract including a urinary bladder, a prostatic urethra, a sphincter and a penile urethra, the indwelling catheter comprising (a) a first tubular member having a distal end and a proximal end; (b) a second tubular member having a length, a distal end and a proximal end, the first and second tubular members having such a diameter for enabling drainage of physiological fluids therethrough; (c) a first inflatable balloon being inflatably attached at a portion of the second tubular member; and (d) a first connecting tube of substantially smaller diameter interconnecting the first and second tubular members to form a gap of a known maximal length between the first and second tubular members, the first connecting tube being in fluid communication with the first inflatable balloon, so as to enable inflating the first inflatable balloon via the first connecting tube; wherein the length of the second tubular member, the known maximal length of the gap and a location of the portion of the second tubular member to which the first inflatable balloon is inflatably attached are selected such that, when positioned in the urinary tract, the balloon is anchored within the urinary bladder, so as to position the second tubular member within the prostatic urethra with its proximal end positioned distally, and in close proximity, to the sphincter, and with its distal end protruding into the urinary bladder, and further so as to position the first tubular member within the penile urethra with its distal end positioned proximally, and close proximity, to the sphincter, while the first connecting tube traverses the sphincter, thereby permitting voluntary control of the sphincter.

According to one embodiments of the invention described below the catheter further comprises a second inflatable balloon inflatably connected to a second portion of the second tubular member and proximally to the first inflatable balloon.

According to still further features in the described preferred embodiments the second inflatable balloon of the catheter is adapted to contain a heated fluid and is therefore usable for thermal treatment (e.g., ablation) procedures.

According to still further features in the described preferred embodiments the first inflatable balloon and the second inflatable balloon of the catheter form a single notched balloon.

According to still further features in the described preferred embodiments the second inflatable balloon of the catheter is in fluid communication with the first connecting tube and thereby inflatable via the first connecting tube.

According to still further features in the described preferred embodiments the second inflatable balloon of the catheter is in fluid communication with a second connecting tube, the second connecting tube interconnecting the first and second tubular members parallely to the first connecting tube, such that inflation of the second inflatable balloon is effected independently of the inflation of the first inflatable balloon.

According to further features in the described preferred embodiments of the present invention the catheter further comprises a detachable guiding element, the guiding element including an elongated tubular member, an elongated inflatable balloon being attached to at least a portion of the length of the elongated tubular member, and a first mechanism for inflating the elongated inflatable balloon. The guiding element being dimensioned for insertion through the first and second tubular members, such that when the elongated inflatable balloon is inflated the catheter is fixed to the guiding element.

According to further features in the described preferred embodiments of the present invention the elongated tubular member of the guiding element is formed with a distal opening, the guiding element further includes a second mechanism for conducting a fluid through the elongated tubular member.

According to further features in the described preferred embodiments of the present invention the elongated tubular member of the guiding element is formed with a closed distal end and further wherein a cavity of the elongated tubular member is in fluid communication with the elongated inflatable balloon via at least one opening.

According to still further features in the described preferred embodiments the guiding element further includes a second inflatable balloon attached to a portion of the elongated tubular member distally to the elongated inflatable balloon and a second mechanism for inflating the second balloon.

According to another aspect of the present invention there is provided an indwelling catheter for insertion into a patient's urinary tract comprising (a) a first tubular member having a distal end and a proximal end; (b) a second tubular member having a length, a distal end and a proximal end, the first and second tubular members having such a diameter for enabling drainage of physiological fluids therethrough; (c) a first inflatable balloon being inflatably attached at a portion of the second tubular member; (d) a first connecting tube of substantially smaller diameter interconnecting the first and second tubular members to form a gap of a known maximal length between the first and second tubular members, the first connecting tube being in fluid communication with the first inflatable balloon, so as to enable inflating the first inflatable balloon via the first connecting tube; and (e) a second inflatable balloon inflatably connected to a second portion of the second tubular member and proximally to the first inflatable balloon.

According to another aspect of the present invention there is provided a guiding element for guiding a catheter comprising an elongated tubular member, a first and elongated inflatable balloon being attached to at least a portion of the length of the elongated tubular member, and a first mechanism for inflating the elongated inflatable balloon. A second inflatable balloon, is provided, attached to a portion of the elongated tubular member distally to the elongated inflatable balloon, and a second mechanism for inflating the second balloon. The guiding element being dimensioned for insertion through the catheter, such that when the elongated inflatable balloon is inflated the catheter is fixed to the guiding element.

According to another aspect of the present invention there is provided a method of positioning an indwelling catheter in a patient's urinary tract, the tract including a urinary bladder, a prostatic urethra, a sphincter and a penile urethra, the method comprising the steps of (a) providing an indwelling catheter including (i) a first tubular member having a distal end and a proximal end; (ii) a second tubular member having a length, a distal end and a proximal end, the first and second tubular members having such a diameter for enabling drainage of physiological fluids therethrough; (iii) a first inflatable balloon being inflatably attached at a portion of the second tubular member; and (iv) a first connecting tube of substantially smaller diameter interconnecting the first and second tubular members to form a gap of a known maximal length between the first and second tubular members, the first connecting tube being in fluid communication with the first inflatable balloon, so as to enable inflating the first inflatable balloon via the first connecting tube; (b) attaching the indwelling catheter to a guiding element; and (c) inserting the indwelling catheter via the guiding element into the patient's urinary tract and positioning the indwelling catheter therein such that the first tubular member engages a portion of the penile urethra proximally to the sphincter, the second tubular member engages the prostatic urethra distally to the sphincter and having its distal end protruding into the urinary bladder, the connecting tube traverses the sphincter.

According to a preferred embodiment positioning the indwelling catheter in the patient's urinary tract is effected by inserting the first inflatable balloon into the urinary bladder, inflating the first inflatable balloon and via the connecting tube pulling the catheter, so as to position the inflatable balloon against a wall of the urinary bladder.

According to another preferred embodiment the method of positioning the indwelling catheter in a patient's urinary tract , further comprises the step of removing the guiding element.

According to another embodiment the method of positioning an indwelling catheter in a patient's urinary tract further comprises the step of deflating the first inflatable balloon.

According to another aspect of the present invention there is provide a method of positioning an indwelling catheter in a patient's urinary tract, the tract including a urinary bladder, a prostatic urethra, a sphincter and a penile urethra, the method comprising the steps of (a) providing an indwelling catheter including (i) a first tubular member having a distal end and a proximal end; (ii) a second tubular member having a length, a distal end and a proximal end, the first and second tubular members having such a diameter for enabling drainage of physiological fluids therethrough; (iii) a connecting element of substantially smaller diameter interconnecting the first and second tubular members to form a gap of a known maximal length between the first and second tubular members; (b) attaching the indwelling catheter to a guiding element, the guiding element including an inflatable balloon attached thereto; and (c) inserting the indwelling catheter via the guiding element into the patient's urinary tract and positioning the indwelling catheter therein such that the first tubular member engages a portion of the penile urethrproximally to the sphincter, the second tubular member engages the prostatic urethra distally to the sphincter and having its distal end protruding into the urinary bladder, the connecting element traverses the sphincter.

According to a preferred embodiment of the present invention the inflatable balloon of the elongated tubular member is positioned at a portion of the guiding element, such that when the guiding element engages the indwelling catheter, the inflatable balloon is inflatably positioned distally to the catheter, whereas positioning the indwelling catheter is effected by inserting the inflatable balloon into the urinary bladder, inflating the inflatable balloon and pulling the guiding element so as to position the inflatable balloon against a wall of the bladder.

According to another aspect of the present invention there is provided a method of urinating a patient following a prostate ablation procedure, the patient's urinary tract including a urinary bladder, a prostatic urethra, a sphincter and a penile urethra the, the method comprising the steps of (a) providing an indwelling catheter including (i) a first tubular member having a distal end and a proximal end; (ii) a second tubular member having a length, a distal end and a proximal end, the first and second tubular members having such a diameter for enabling drainage of physiological fluids therethrough; (iii) an inflatable balloon being inflatably attached at a portion of the second tubular member; (iv) a connecting tube of substantially smaller diameter interconnecting the first and second tubular members to form a gap of a known maximal length between the first and second tubular members, the first connecting tube being in fluid communication with the first inflatable balloon, so as to enable inflating the first inflatable balloon via the first connecting tube; (b) attaching the indwelling catheter to a guiding element; (c) inserting the indwelling catheter via the guiding element into the patient's urinary tract and positioning the indwelling catheter therein such that the first tubular member engages a portion of the penile urethra proximally to the sphincter, the second tubular member engages the prostatic urethra distally to the sphincter and having its distal end protruding into the urinary bladder, the connecting tube traverses the sphincter, and (d) removing the guiding element.

According to another aspect of the present invention there is provided a method of dilating a prostatic urethra of a patient following a prostate ablation procedure, while at the same time urinating the patient, the method comprising the steps of (a) inserting a drainage catheter into a urinary tract of the patient, the catheter including an inflatable balloon; (b) positioning the catheter within the urinary tract such that the inflatable balloon engages the prostatic urethra; and (c) inflating the inflatable balloon, so as to dilate the prostatic urethra.

According to an embodiment of the present invention the method of dilating a prostatic urethra, further comprises the steps of deflating said inflatable balloon and removing said drainage catheter.

According to another aspect of the present invention there is provided a method of ablating a prostatic urethra of a patient during a prostate ablation procedure, while at the same time implanting a drainage catheter in a urinary tract of the patient for urinating the patient following the ablation procedure, the method comprising the steps of (a) inserting an ablating-drainage catheter into a urinary tract of the patient, the catheter including an inflatable balloon; (b) positioning the catheter within the urinary tract such that the inflatable balloon engages the prostatic urethra; (c) inflating the inflatable balloon with a heated fluid so as to ablate the prostatic urethra; and (d) leaving the ablating-drainage catheter in the urinary tract for urinating the patient following the ablation procedure.

According to a preferred embodiment of the present invention, the inflatable balloon of the catheter is maintained inflated for dilating the prostatic urethra following the ablation procedure.

According to an embodiment of the present invention the method of ablating a prostatic urethra and at the same time urinating a patient further comprises the steps of removing the ablating-drainage catheter.

Further according to an embodiment of the present invention, there is provided an indwelling catheter for insertion into a patient's urinary tract, the tract including a urinary bladder, a prostatic urethra, a sphincter and a penile urethra, the indwelling catheter comprising (a) a first tubular member having a distal end and a proximal end; (b) a second tubular member having a length, a distal end and a proximal end, the first and second tubular members having such a diameter for enabling drainage of physiological fluids therethrough; (c) a first inflatable balloon inflatably connected to a portion of the second tubular member; and (d) a first connecting tube of substantially smaller diameter interconnecting the first and second tubular members to form a gap of a known maximal length between the first and second tubular members, the first connecting tube being in fluid communication with the first inflatable balloon, so as to enable inflating the first inflatable balloon via the first connecting tube, wherein the length of the second tubular member, the known maximal length of the gap and a location of the portion of the second tubular member to which the first inflatable balloon is inflatably attached are selected such that, when positioned in the urinary tract, the balloon engages the prostatic urethra and the second tubular member has its proximal end positioned distally, and in close proximity, to the sphincter, and its distal end protruding into the urinary bladder, whereas the first tubular member engages the penile urethra having its distal end positioned proximally, and is in close proximity, to the sphincter, while the first connecting tube traverses the sphincter, thereby permitting voluntary control of the sphincter.

Preferably, the catheter of further comprising (e) a detachable guiding element, the guiding element including (i) an elongated tubular member; (ii) an elongated inflatable balloon being attached to at least a portion of the length of the elongated tubular member; (iii) a first mechanism for inflating the elongated inflatable balloon, the guiding element being dimensioned for insertion through the first and second tubular members, such that when the elongated inflatable balloon is inflated the catheter is fixed to the guiding element; and (iv) a second inflatable balloon attached to a portion of the elongated tubular member distally to the elongated inflatable balloon and a second mechanism for inflating the second balloon, wherein the second inflatable balloon serves for positioning the catheter in the urinary tract of the patient.

The present invention successfully addresses the shortcomings of the presently known configurations by providing a catheter and guide system with simple means for accurately positioning and securely anchoring the catheter within a patient's urinary tract, such that voluntary biological control of urination is maintained. Furthermore, the catheter of the present invention can also be used to both ablate and dilate a patients prostatic urethra, while at the same time allowing the urination of the patient.

### BRIEF DESCRIPTION OF THE DRAWINGS

The invention herein described, by way of example only, with reference to the accompanying drawings, wherein:
FIG. 1a is a longitudinal cross sectional view of a catheter including a positioning and anchoring inflatable balloon according to the present invention;
FIG. 1b is a longitudinal cross sectional view of a guiding element including an elongated inflatable balloon according to the present invention;
FIG. 1c is a cross sectional view of an elongated tubular element of the guiding element shown in Figure 1b;
FIG. 1d is a longitudinal cross sectional view of the catheter shown in Figure 1a and a guiding element including an elongated inflatable balloon attached thereto according to the present invention;
FIG. 1e is a cross sectional view of an elongated tubular element of the guiding element of Figure 1d;
FIG. 2a is a longitudinal cross sectional view of a catheter according to another aspect of the present invention which includes two integrated inflatable balloons;
FIG. 2b is a longitudinal cross sectional view of the catheter of Figure 2a and a guiding element attached thereto according to the present invention;
FIG. 3a is a longitudinal cross sectional view of a catheter according to yet another embodiment of the present invention, which includes two separated inflatable balloons;
FIG. 3b is a longitudinal cross sectional view of the catheter and of Figure 3a and a guiding element attached thereto according to the present invention;
FIG. 3c is a cross sectional view along line A-A in Figure 3b;
FIG. 3d is a cross sectional view along line B-B in Figure 3b;
FIG. 4a is a longitudinal cross sectional view of a prior art catheter attached to a guiding element including two inflatable balloons according to the present invention;
FIG. 4b is a cross sectional view along line A-A in Figure 4a;
FIG. 5 is a longitudinal cross sectional view of a catheter according to the prior art; and
FIG. 6 is a longitudinal cross sectional view of a catheter according to the present invention, which includes two integrated inflatable balloons and which is supplemented with a Tiemann pattern tip.

### DESCRIPTION OF THE PREFERRED EMBODIMENTS

The present invention is of an indwelling urethral catheter which can be used for urinating a patient after a surgical procedure such as TURP or a non-surgical procedure such as thermal ablation of the prostatic urethra. Specifically the catheter of the present invention can be used for both thermal ablation and subsequent urination of the patient. The present invention is further of a catheter guiding element and catheter insertion and positioning methods to be used along with the catheter of the present invention.

The principles and operation of an indwelling urethral catheter, guiding element and methods according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

As used herein in the specification and in the claims section that follows, and in relation to the urinary tract positioning of the catheter or guiding element according to the present invention, the terms "distal" and "distally" denote towards the urinary bladder and the terms "proximal" and "proximally" denote towards the penile urethra.

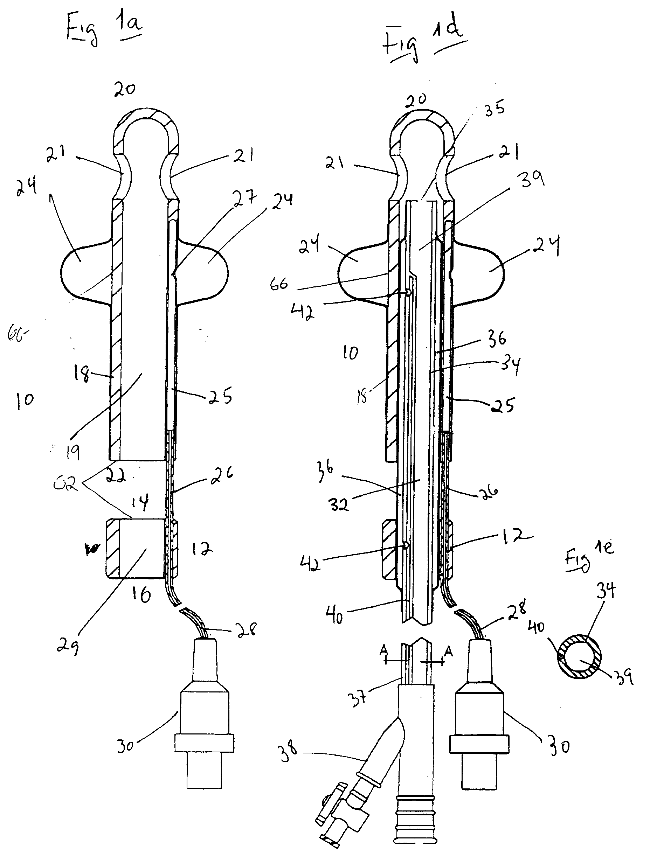

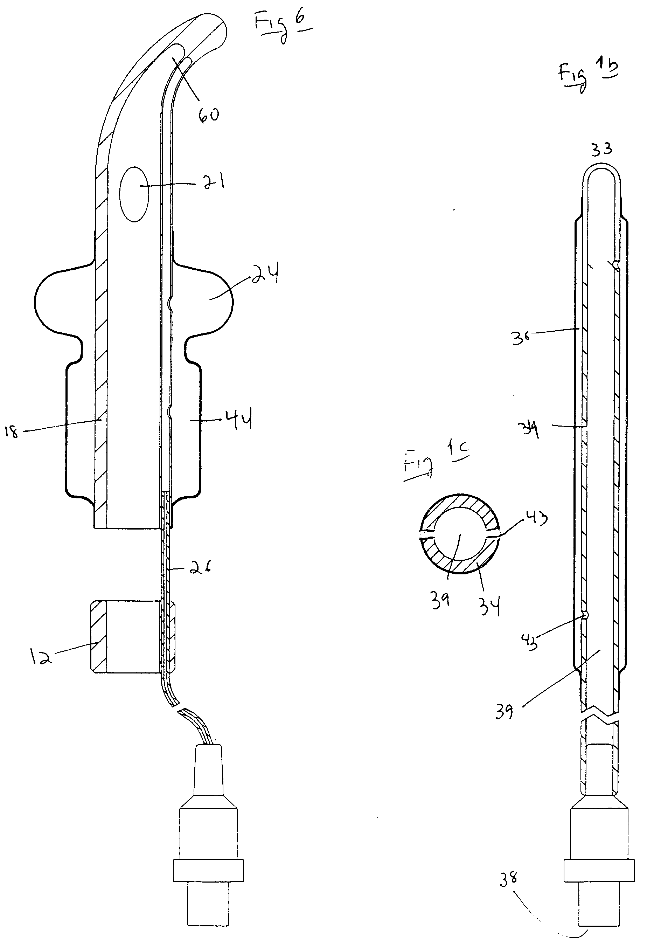

Referring now to the drawings, Figures 1a, 1d, 2a-b, 3a-b and 6 illustrate an indwelling urethral catheter according to some preferred embodiments of the present invention, which is referred to hereinunder as catheter **10**. As further detailed hereinunder, catheter **10** is designed for insertion into a patient's urinary tract and serves primarily for urinating the patient.

Catheter **10** includes a first tubular member **12** having an inner lumen **29**. Member **12** has a distal open end **14** and a proximal open end **16**. When catheter **10** is implanted in the patient's urinary tract, member **12** is designed to engage the distal portion of the patient's penile urethra, just proximally to the sphincter.

Catheter **10** further includes a second tubular member **18** having an inner lumen **19**. Member **18** has a distal end **20** and an open proximal end **22**. Distal end **20** is either open or formed with at least one opening **21**. A distal portion of tubular member **18**, which includes end **20** and opening **21**, can feature any one of a variety of patterns known in the art and which serve for facilitating guidance of, and/or urination through, catheter **10**, such as, but not limited to, Tiemann pattern (see Figure 6, at **60**), Couvelaire pattern, Dufour pattern, Mercier pattern, a whistle tip pattern and a cylindrical pattern. These patterns are further described in an endourology catalog by RUSCH Incorporated of 2450 Meadowbrook Parkway Duluth, Georgia 30136, and in http://www.ruesch.de, the web site thereof.

When catheter **10** is implanted, member **18** engages the prostatic urethra, having its proximal end **22** positioned just distally to the distal end of the sphincter and its distal end **20**, as well as opening **21**, within the bladder of the patient.

Thus, members **12** and **18** serve for draining the bladder under the voluntary control of the sphincter, which is not engaged or traversed by neither member **12** nor member **18**. To this end, fluids from the bladder are directed via member **18** to the sphincter, and when the sphincter muscle is contracted, and as a result the sphincter is dilated, these fluids pass therethrough to member **12** and then discarded through the proximal end of the penile urethra.

Second tubular member **18** is preferably designed with several lengths, preferably between **2** and **14** centimeters, more preferably, between **3** and **12** centimeters, most preferably between **4** and **10** centimeters, so as to fit prostatic urethras of different patients preferably between **2** and **6** cm in length. While restrictions are imposed on the length of member **18**, which should engage the entire length of the prostatic urethra and protrude into the bladder, no such length restrictions are imposed on member **12**, which is shown in the drawings to be shorter than member **18**, yet in reality can be substantially longer. The length of member **12** can range from about one centimeter or less to about 8-10 centimeters or more.

The inner diameter of members **12** and **18** is selected wide enough so as to enable free passage of body fluids therethrough. Inner diameters above about 5 millimeters are preferred. The outer diameter of members **12** and **18** is preferably about 6-9 millimeters. Members **12** and **18** are typically made of a resilient and hypoallergenic polymeric material, such as, but not limited to, polyethylene, polypropylene, polyurethane, polyvinyl chloride (PVC) and silicon.

First **12** and second **18** tubular members are interconnected therebetween by a connecting tube **26**. Tube **26** typically serves three functions, one of which is to form a gap **62** of a known (limited) maximal length between members **12** and **18**. The other functions of tube **26** are further addressed hereinunder. When catheter **10** is positioned within the urinary tract of the patient, tube **26** traverses the sphincter. The length of gap **62** is selected so as to enable the sphincter to be free of any portions of members **12** and **18**. Tube **26** is of a substantially smaller diameter as compared with the diameters of members **12** and **18** (both internal and external). Tube's **26** external diameter is selected such that it does not interfere with the hermetic closure of the sphincter, and as such, allows the patient voluntary control over urination via the sphincter. Furthermore, retaining catheter **10** in position is generally achieved by the sphincter which prevents the longitudinal dislocation of members **12** and **18** when closed.

Connecting tube **26** extends through member **12** and has sufficient length so as to extend the length of the urethra, through the penis and have its proximal end outside the body of the patient. According to a preferred embodiment, the portion of connecting tube **26** that traverses member **12** is engaged within the wall of member **12**. In fact, according to one embodiment, that portion of tube **26** is directly formed by a channel present in the wall of member **12**.

The above described configuration is common to all of the embodiments of catheter **10** according to the present invention as further detailed hereinunder.

According to a preferred embodiment of the present invention, catheter **10** further includes an inflatable balloon **24**, which is shown inflated in the drawings. Inflatable balloon **24** is attached at a portion **66** of second tubular member **18** and is positioned proximally to distal end **20** and opening **21**.

Balloon **24** serves for positioning catheter **10** in the patient's urinary tract. To this end, inflatable balloon **24** is inserted in its deflated form into the patients bladder. It is thereafter inflated so as to anchor catheter **10** in place. To this end, when inflated, balloon **24** is of substantial girth, such that it forms a steepled donut shape, substantially perpendicular to, and surrounding, second tubular member **18**. When inflated, inflatable balloon **24** is larger in diameter than the opening of the patient's bladder, such that subsequent pulling of catheter **10** in a proximal direction via, for example, proximal end **28** of connecting tube **26**, anchors balloon **24** against the bladder's inner wall and positions first **12** and second **18** tubular members and connecting tube **26** as described hereinabove to allow voluntary control over urination.

Retaining catheter **10** in position is generally achieved by the sphincter which prevents the longitudinal dislocation of members **12** and **18** when closed. However, a possibility exists, in which, members **12** and **18** will dislocate while the sphincter is dilated (i.e., during voluntary urination). To prevent such undesired dislocation, balloon **24** is preferably retained in its inflated form also during service.

It will be appreciated that the prior art catheter of Boston Scientific, which is described in the Background section hereinabove and is shown in Figure 5, can dislocate when the patient urinates, due to dilation of the sphincter during urination.

Thus, catheter **10** of the present invention provides a second and effective means, unaffected by urination of the patient, for retaining catheter **10** appropriately positioned in the urinary tract.

An additional function attributed to connecting tube **26** involves the inflation/deflation of balloon **24**. To this end, connecting tube **26** traverses a distal portion of member **18**. The portion of connecting tube that traverses the distal portion of member **18** is preferably engaged within the wall of member **18**. In fact, according to one embodiment, that portion of tube **26** is formed by a channel **25** present in the wall of member **18**. Thus, tube **26** can be formed by tubular portions in part, and channels in part.

According to the present invention, tube **26** is in fluid communication with inflatable balloon **24**. The fluid communication between tube **26** and balloon **24** is formed either by directly connecting a tubular portion of tube **26** to balloon **24**, or preferably by an opening **27** formed in the wall of member **18** forming a fluid communication between channel **25** and balloon **24**. Thus, a direct fluid passage exists between tube **26** and balloon **24**.

An adapter **30** is preferably connected at proximal end **28** of tube **26**, to enable the connection of an inflation device, such as, but not limited to, a syringe or a pump, thereto, so as to enable inflation and/or deflation of inflatable balloon **24**. Deflation of balloon **24** can also be performed passively. Preferably inflation and/or deflation of balloon **24** is achieved with air but other fluids, such as water, can also be used, as well as other gasses, such as nitrogen.

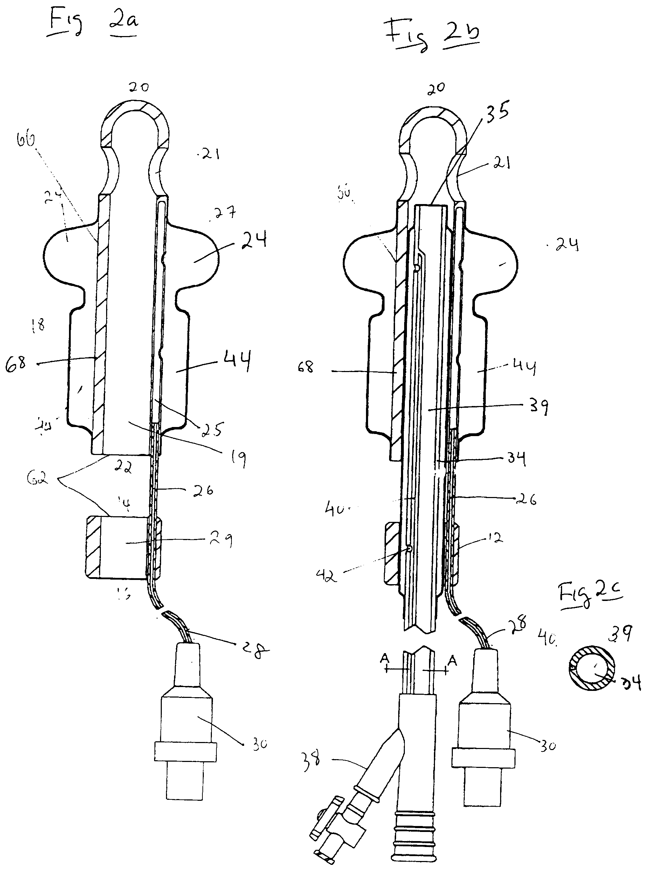

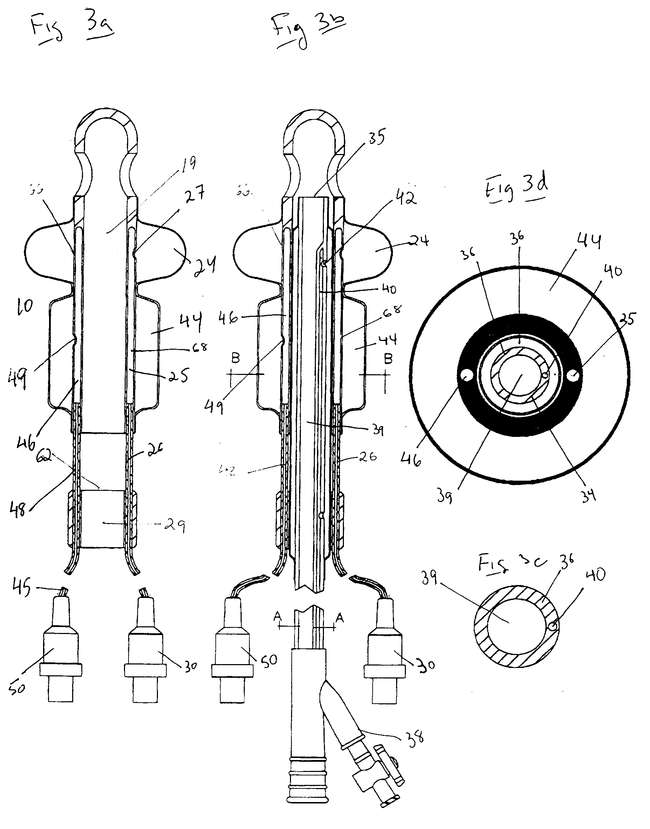

According to another preferred embodiment of the present invention, and as specifically shown in Figures 2a-b and 3a-b, catheter **10** further or alternatively includes an additional inflatable balloon **44**, which is shown inflated in the drawings. Balloon **44** is inflatably connected to a second portion **68** of second tubular member **18** and proximally to balloon **24**, in cases the latter is present.

When catheter **10** is appropriately positioned within the urinary tract of the patient, balloon **44** is designed to engage the prostatic urethra. Balloon **44** can serve several functions, as further delineated in detail below, including anchorage of catheter **10**, facilitated removal of catheter **10**, dilation of the prostatic urethra, ablation of the prostatic urethra and/or drug delivery thereto. To this end, when inflated, balloon **44** is more elongated, and narrower as is compared to balloon **24**.

Thus, typically, balloon **44** is inflated following the positioning of catheter **10** and is utilized for the dilation of the prostatic urethra following a non surgical prostatic procedure such as, but not limited to, thermal ablation thereof. Inflated balloon **44** is retained in its inflated form until the prostatic urethra retains a dilated diameter due to scarring of tissue surrounding inflated balloon **44**, at which time, and prior to the removal of catheter **10**, balloon **44** is deflated.

It will be appreciated that, balloon **44** of catheter **10** can be used for thermal ablation of a prostatic urethra. In this case, catheter **10** serves both as a thermal ablation catheter and then also as a drainage catheter, without the need of catheter replacement. To this end, balloon **44** and additional components of catheter **10** are constructed from heat resistant materials, such that a heated fluid can be conducted therethrough. Preferred materials will withstand temperatures of between 55 °C to 80 °C, examples include, but not limited to, PVC, silicon and polyurethane.

Following the thermal ablation procedure, balloon **44** is maintained inflated for dilation of the prostatic urethra as further described hereinabove. Thus, the present invention makes possible the use of a single device for both the thermal ablation and urination of the patient. U.S. Pat. Nos. 5,257,977; 5,549,559; and 5,492,529, which are incorporated by reference as if fully set forth herein, provide further details relating to ablation catheters directed at ablating a prosthetic urethra.

Yet, it will be appreciated that balloon **44** of catheter **10** can also serve for the diffusional release of a medicament such as, but not limited to, non-steroidal anti-inflammatory drugs typically used in post thermal ablation treatments. In this embodiment of the present invention balloon **44** is manufactured permeable to the medicament or medicaments of choice, such that a liquid carrying the medicament(s), dissolved or in suspension, can be conducted to balloon **44** and delivered to the body of the patient therethrough. Balloon **44** can be inflated with the medicament containing fluid such that it contacts the ablated prostatic urethral, and thus diffusional drug transfer is facilitated. U.S. Pat. Nos. 5,282,785; and 5,800,392, which are incorporated by reference as if fully set forth herein, provide further details relating to balloon catheter based drug delivery.

According to one configuration of the present invention, each of balloons **24** and **44** is independently inflated (Figure 3a-b). To this end an additional connecting tube **48**, which substantially parallels connecting tube **26**, is employed, and is provided in fluid communication with balloon **44**. Connecting tube **48** establishes fluid communication with an adapter **50**, provided at a proximal end **45** thereof and which is located outside the patient's body, through channel **46** formed in tubular member **18**. Channel **46** is in fluid communication with balloon **44** through at least one opening **49**. Inflation and deflation of balloon **44** is achieved, according to this configuration, via an inflating/deflating device, such as, but not limited to, a syringe or a pump connectable to adapter **50**.

However, according to another configuration of the present invention, balloons **24** and **44** are co-inflatable. This is achieved either by forming a fluid communication between balloon **44** and tube **26** (Figure 2a-b) and/or by integrating balloons **24** and **44** into preferably a single notched balloon (Figure 2a-b), although other non-isometric shapes are also envisaged for such a balloon. In each of these cases, the degree to which each of balloons **24** and/or **44** inflates under predetermined pressure, is dictated by its specific characteristics. Typically balloon **24** is selected the first to substantially inflate, so as to serve in the process positioning catheter

**10** as described above. Guiding and positioning catheter **10** as herein described is effected according to preferred embodiments of the present invention via a guiding element, which is shown in Figures 1 b-d, 2b-c, 3b-d and 4a at **32**.

Guiding element **32** includes an elongated tubular member **34** which includes a lumen **39**, and which is formed with a closed (Figure 1b) or opened (Figures 1d, 2b and 3b) distal end **33** and an preferably an open proximal end **37**. Guiding element **32** further includes an elongated inflatable balloon **36** inflatably attached to member **34**. Balloon **36** is inflatable either via openings **43** formed in the wall of member **34** connecting lumen **39** thereof with balloon **36** (Figure 1b), or by a dedicated channel **40** (Figures 1d, 2b-c and 3b-d) formed in the wall of member **34** and opens to balloon **36**. A double headed adapter **38** is preferably provided in fluid communication with either lumen **39** and/or channel **40** and is used for connecting an inflating device for inflating/deflating balloon **36** and/or connecting a perfusing device for perfusing liquids in an out of the body of the patient.

The diameter of guiding element **32** is selected so as to enable its insertion through members **12** and **18** of catheter **10** when balloon **36** is deflated. Inflating balloon **36** ensures fixation of element **32** and catheter **10**, such that via element **32**, which is constructed of sufficient length and rigidity, catheter **10** is guided to its appropriate position within the urinary tract of the patient, as further detailed hereinabove.

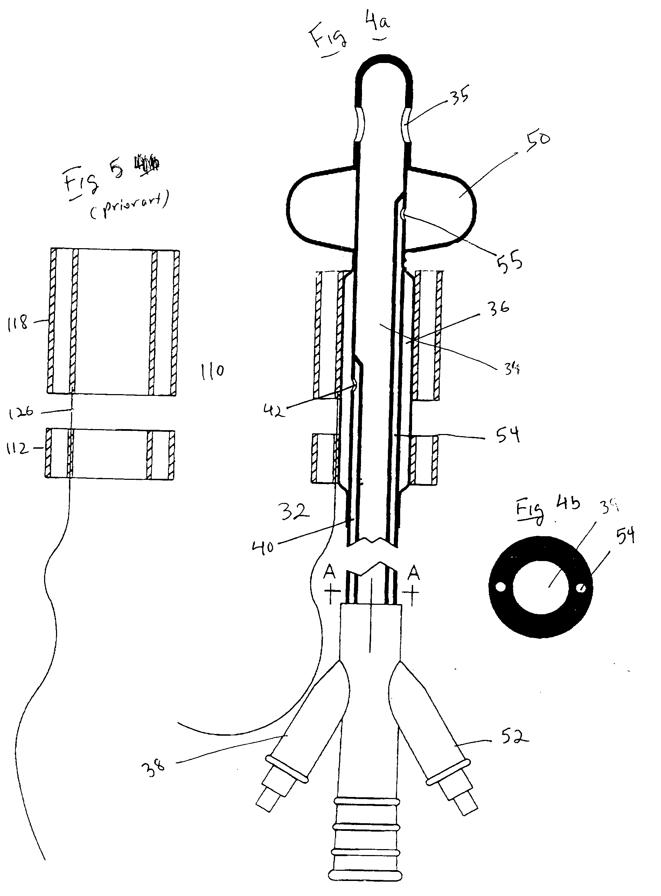

As shown in Figure 4a, according to one embodiment of the present invention, guiding element **32** further includes an additional or alternative balloon **50**, located distally to balloon **36**, if the latter is present. Balloon **50** is shaped and functions similarly to balloon **24** of catheter **10**. This configuration of element **32** is directed at insertion and positioning of catheters lacking a bladder positioning balloon (e.g., balloon **24**). Such catheters include the configuration of catheter **10** according to the present invention in which only balloon **44** is employed, or prior art configurations in which no balloons are employed altogether, such as shown in the prior art catheter of Figure 5, in which two tubular members **112** and **118** are interconnected by a connecting element, such as a thread **126**.

Balloon **50** is in fluid communication with an adapter **52** through a channel **54** formed in the wall of member **34** and which opens to balloon **50** at opening **55**, such that balloon **50** is inflated independently of balloon **36** (when the latter is present) via adapter **52**. Like balloon **24** of catheter **10**, balloon **50** serves for positioning catheter **10** within the patient's urinary tract.

To this end, an indwelling catheter, e.g., catheter **110**, an example of which is shown in Figure 5, or catheter **10** according to the present invention, is attached to guiding element **32** by first inserting guiding element **32** therethrough and then inflating balloon **36** thereof. Then, guiding element **32** is guided through the urinary tract of the patient, until balloon **50** is within the bladder of the patient. Thereafter, balloon **50** is inflated and thereby anchors element **32** within the body of the patient. Subsequent pulling of element **32** such that balloon **50** is in contact with the wall of the bladder of the patient ensures accurate positioning of catheter **10** or **110** within the urinary tract thereof. Deflating both balloons **36** and **50**, enables to retrieve element **32**, while catheter **10** or **110** is retained in place.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An indwelling catheter for insertion into a patient's urinary tract, the tract including a urinary bladder, a prostatic urethra, a sphincter and a penile urethra, the indwelling catheter comprising:
(a) a first tubular member having a distal end and a proximal end;
(b) a second tubular member having a length, a distal end and a proximal end, said first and second tubular members having such a diameter for enabling drainage of physiological fluids therethrough;
(c) a first inflatable balloon being inflatably attached at a portion of said second tubular member;
(d) a first connecting tube of substantially smaller diameter interconnecting said first and second tubular members to form a gap of a known maximal length between said first and second tubular members, said first connecting tube being in fluid communication with said first inflatable balloon, so as to enable inflating said first inflatable balloon via said first connecting tube;
wherein said length of said second tubular member, said known maximal length of said gap and a location of said portion of said second tubular member to which said first inflatable balloon is inflatably attached are selected such that, when positioned in the urinary tract, said balloon is anchored within the urinary bladder, so as to position said second tubular member within the prostatic urethra with its proximal end positioned distally, and in close proximity, to the sphincter and with its distal end protruding into the urinary bladder, and further so as to position said first tubular member within the penile urethra with its distal end positioned proximally, and in close proximity, to the sphincter, while said first connecting tube traverses the sphincter, thereby permitting voluntary control of the sphincter.

2. Use of an indwelling catheter, while positioning it in a patient's urinary tract, the tract including a urinary bladder, a prostatic urethra, a sphincter and a penile urethra, comprising:
(a) providing an indwelling catheter including:
i. a first tubular member having a distal end and a proximal end;
ii. a second tubular member having a length, a distal end and a proximal end, said first and second tubular members having such a diameter for enabling drainage of physiological fluids therethrough;
iii. a first inflatable balloon being inflatably attached at a portion of said second tubular member; and
iv. a first connecting tube of substantially smaller diameter interconnecting said first and second tubular members to form a gap of a known maximal length between said first and second tubular members, said first connecting tube being in fluid communication with said first inflatable balloon, so as to enable inflating said first inflatable balloon via said first connecting tube;
(b) attaching said indwelling catheter to a guiding element; and
(c) shaping said catheter so that it can be inserted via said guiding element into a patient's urinary tract and that can be positioned therein, in a way that said first tubular member engages a portion of the penile urethra proximally to the sphincter, said second tubular member being so structured as to engage the prostatic urethra distally to the sphincter and as to have its distal end protruding into the urinary bladder, said connecting tube traversing said sphincter.

3. The invention according to claims 1 or 2, wherein said catheter further comprises a second inflatable balloon inflatably connected to a second portion of said second tubular member and proximally to said first inflatable balloon.

4. The invention of claim 1, wherein said second inflatable balloon is adapted to contain a heated fluid and is therefore usable for thermal treatment procedures.

5. The invention of claim 3, wherein said first inflatable balloon and said second inflatable balloon form a single notched balloon.

6. The invention of claim 3, wherein said second inflatable balloon is in fluid communication with said first connecting tube and thereby inflatable via said first connecting tube.

7. The invention of claim 3, wherein said second inflatable balloon is in fluid communication with a second connecting tube, said second connecting tube interconnecting said first and second tubular members parallel to said first connecting tube, such that inflation of said second inflatable balloon is effected independently of inflation of said first inflatable balloon.

8. The invention according to claim 1, 2 or 3, where in said catheter further comprises a detachable guiding element, said guiding element including:
(i) an elongated tubular member;
(ii) a first elongated inflatable balloon being attached to at least a portion of the length of said elongated tubular member; and
(iii) a first mechanism for inflating said elongated inflatable balloon; said guiding element being dimensioned for insertion through said first and second tubular members, such that when said elongated inflatable balloon is inflated said catheter is fixed to said guiding element.

9. The catheter of claim 8, wherein said guiding element further includes a second inflatable balloon attached to a portion of said elongated tubular member distally to said elongated inflatable balloon and a second mechanism for inflating said second balloon.

10. The catheter of claim 8, wherein said elongated tubular member of said guiding element is formed with a distal opening, said guiding element further includes a second mechanism for conducting a fluid through said elongated tubular member.

11. The catheter of claim 8 or 9, wherein said elongated tubular member of said guiding element is formed with a closed distal end and further wherein a cavity of said elongated tubular member is in fluid communication with said elongated inflatable balloon opening via at least one opening.

12. The catheter of claim 9, wherein said elongated tubular member of said guiding element is formed with a distal opening, said guiding element further includes a third mechanism for conducting a fluid through said elongated tubular member.

13. The catheter of claim 8 or 9, wherein said elongated tubular member of said guiding element is formed with a closed distal end and further wherein a cavity of said elongated tubular member is in fluid communication with said elongated inflatable balloon via at least one opening.

14. The use of the catheter according to claim 2, wherein said indwelling catheter is structured so as to allow the catheter to be inserted in a patients urinary tract in a way that said first inflatable balloon is inserted into the urinary bladder, said first inflatable balloon is inflated and via said connecting tube said catheter is pulled, said inflatable balloon being structured so that when inflated, it inflates against a wall of the urinary bladder.

15. The use of the catheter according to claim 2 or 8, wherein said indwelling catheter is structured so as to be attached to said guiding element by inserting said guiding element through said first and second tubular members, said elongated inflatable balloon is inflated via said first mechanism, thereby fixing said indwelling catheter to said guiding element.
